# EUROPÄISCHE PATENTSCHRIFT

(11) **EP 0 505 527 B1**
(45) Veröffentlichungstag und Bekanntmachung des Hinweises auf die Patenterteilung: **19.07.1995**
(21) Anmeldenummer: 91916446.7
(22) Anmeldetag: 13.09.1991
(51) Int. Cl.: A61F 5/04, A61F 5/01, A61F 13/06

(54) **VORRICHTUNG ZUR UMSCHLIESSENDEN FIXIERUNG VON EXTREMITÄTEN**
DEVICE FOR THE COMPREHENSIVE IMMOBILISATION OF LIMB ENDS
DISPOSITIF POUR LA FIXATION ENVELOPPANTE D'EXTREMITES

(30) Priorität: 13.09.1990 DE 4029120
(43) Veröffentlichungstag der Anmeldung: 30.09.1992
(73) Patentinhaber: Habermeyer, Peter, Dr., D-70184 Stuttgart (DE)
(72) Erfinder: HABERMEYER, Peter, D-70184 Stuttgart (DE); LEDERER, Stefan, D-8000 München 40 (DE); HASSLER, Andreas, D-8032 Gräfelfing (DE)
(74) Vertreter: Richter, Werdermann & Gerbaulet
(86) Internationale Anmeldenummer: EP9101744
(87) Internationale Veröffentlichungsnummer: WO9204880

(56) Entgegenhaltungen:
- EP-A- 0 355 930
- WO-A-86/05087
- DE-A- 3 228 753
- US-A- 3 955 565
- US-A- 4 693 239
- US-A- 4 771 768
- US-A- 4 919 118

## Beschreibung

Die Erfindung betrifft eine Vorrichtung zur umschließenden Fixierung von Extremitäten und Extremitätenbereichen, insbesondere zur Behandlung von Extremitätenfrakturen im Bereich des Unterschenkels, bestehend aus einer die Extremität umschließenden, zu einer Hülse verformbaren, vakuumdicht ausgebildeten, evakuierbaren und mit einem Ventil versehenen Manschettenkissen, in dessen zwischen den beiden Kissenwänden gelegenem Innenraum eine Vielzahl von losen Füllkörperteilchen vorgesehen ist und das durch Evakuierung zu einem in sich steifen, stabilen Gebilde verfestigbar ist, und aus einer äußeren, das unter Vakuum stehende Manschettenkissen stabilisierenden Hülse, die in etwa die Form des Unterschenkels aufweist und schalenartig ausgebildet ist.

Zur Behandlung von Extremitätenfrakturen werden üblicherweise Gipsverbände oder deren moderne Varianten in Form von Kunststoffverbänden angelegt. Das Anlegen derartiger Verbände erfordert nicht nur ein erhebliches Geschick und einen entsprechenden Aufwand für das Personal, sondern führt häufig auch dazu, daß durch Kanten oder Vorsprünge an der Innenseite des Gips- oder Plastikverbandes für den Patienten äußerst störende Druckstellen entstehen. Nach der Reposition der jeweiligen Fraktur ist der Patient der schmerzhaften Phase des Nachwickelns der Binden und des Wartens auf das Abbinden des Gipses ausgesetzt. Schließlich ist es bei den bekannten Gips- oder Plastikverbänden im Regelfall erforderlich, einen Wechsel dieses Gips- oder Plastikverbandes vorzunehmen, wenn es im Laufe der Frakturbehandlung zu einer Abschwellung der betreffenden Gliedmaße kommt. Auch dies ist ein aufwendiger und für den Patienten wiederum unangenehmer Vorgang.

Durch die EP-A-0 355 930 ist eine Vorrichtung zur umschließenden Fixierung von Extremitäten bekannt, bei der ein doppelwandiges, zu einer Hülse verformbares, vakuumdicht ausgebildetes und mit einem Evakuierungsventil versehenes Manschettenkissen verwendet wird, in dessen zwischen den beiden Wänden gelegenem Innenraum eine Vielzahl von losen Füllkörperteilchen vorgesehen ist und das durch Evakuierung zu einem in sich steifen, stabilen Gebilde verfestigbar ist. Zum Schutz des unter Vakuum stehenden Gebildes und zur weiteren Stabilisierung erfolgt die Anordnung des Manschettenkissens in einer äußeren Kunststoffhülse, die in etwa die Form des Unterschenkels aufweist und schalenartig ausgebildet oder so flexibel ist, daß über einen ventralen Schlitz ein Anlegen möglich ist. An der Innenseite der äußeren Kunststoffhülse ist eine hart-elastische Polsterung vorgesehen, welche die Zwischenräume zwischen der verfestigten Innenhülse und der Außenhülse ausfüllt und zugleich als Stoßpuffer bei Aufprallbelastung wirkt und z. B. beim Gehen Widerstand leistet. Die bei dieser Vorrichtung verwendete Außenhülse wird nicht gebildet von zwei gegeneinander verspannbaren Schalenteilen, die die zu umschließende Extremität umgeben. Hinzu kommt, daß das Fußteil ungelenkig am Schaftteil des rückwärtigen Schalenteiles der Vorrichtung angeformt ist, so daß keine Möglichkeit besteht, nach erfolgtem Anlegen der Vorrichtung die Winkelstellung zwischen dem Fuß und dem Unterschenkel zu verändern, d. h. es besteht keine Möglichkeit, von der Normalwinkelstellung von 90° des Fußes zum unteren Gelenk in eine Winkelstellung von 120° zu verändern, um die Spitzfußstellung des Fußes zum Unterschenkel einstellen zu können. Hinzu kommt, daß durch die Vollwandigkeit des verwendeten Manschettenkissens keine Luftzirkulations möglich ist, so daß es zu Schwitzwasserbildung und Wärmestaus kommen kann, die zu einer Verzögerung des Heilprozesses führen.

Die US-A-4 919 118 beschreibt eine Vorrichtung zur Fixierung von Extremitäten, bei der die Außenhülse von einem Schalenkörper gebildet wird, an den ein Fußteil angelegt ist. Den vorderen Verschluß bildet ein schalenförmiger Körper, der die Funktion eines Polsterkissens aufweist. Beide Schalenteile werden gegeneinander verspannt, um eine die Extremität umschließende Hülse zu erhalten. Der Innenraum der Hülse ist ausgepolstert, jedoch ein zwischen den Schalenteilen und der Extremität angeordnetes Manschettenkissen, das evakuierbar ist und in seinem Innenraum eine Vielzahl von relativ zueinander beweglichen, insbesondere kleinkörnigen Füllkörpern aufweist, um durch Evakuierung ein in sich steifes, stabiles Gebilde zu erhalten, ist bei dieser bekannten Vorrichtung nicht vorgesehen.

Aufgabe der Erfindung ist es, eine Vorrichtung der eingangs angegebenen Art in der Weise auszubilden, daß die geschilderten Nachteile von Gips- und Plastikverbänden vermieden werden können und eine einfach und schnell vornehmbare, zu keinerlei Druckstellen führende umschließende Fixierung von Extremitäten möglich ist, die praktisch unverzüglich wirksam ist, einfach gelöst und sofort wieder neu angelegt werden kann, wobei gleichzeitig ein Belüftungs- und Klimasystem geschaffen werden soll.

Diese Aufgabe wird nach der Erfindung durch die im Anspruch 1 angegebenen Merkmale gelöst.

Die eng und passend an den zu behandelnden Gliedabschnitt anlegbaren Kissen werden bei einer innerhalb kurzer Zeit durchführbaren Evakuierung in der während des Anlegevorgangs geschaffenen Form hart und formstabil, so daß in Verbindung mit den harten Schalenteilen eine sehr feste, an die jeweilige Extremität gut anmodellierte Hülse entsteht, die im Bereich der Haut keinerlei Druckstellen erzeugen kann, da bei der Verfestigung dieses Gebildes kein radial nach innen gerichteter Druck entsteht und an der Innenseite auch keine Kanten oder Vorsprünge gebildet werden können.

Die an den Längsrandbereichen von Fußteil und Schaftteil vorgesehenen Verschlußorgane, die aus streifenförmigen Klettverschlußteilen bestehen können, aber bevorzugt aus Rastvorrichtungen mit ineinanander steckbaren Rastelementen bestehen, sorgen sehr einfach und schnell für den vor dem Evakuieren notwendigen leichten Anpreßdruck der Kissen an das umschlossene Körperteil. Auf diese Weise läßt sich eine Hülse absolut korrekter Paßform erzeugen und zunächst über das Verschlußorgan festlegen. Nach erfolgter Evakuierung liegt dann eine Hülse vor, die in sich verfestigt in ihrer Form stabilisiert ist.

Weitere vorteilhafte Ausgestaltungen der Erfindung werden in den Unteransprüchen angegeben.

Die Erfindung wird nachfolgend anhand eines Beispiels unter Bezugnahme auf die Zeichnung näher erläutert; in dieser zeigt:
- Fig. 1: eine Seitenansicht einer Vorrichtung,
- Fig. 2: eine Draufsicht auf eine Vorrichtung,
- Fig. 3: einen Vertikalschnitt durch die Vorrichtung,
- Fig. 4: einen Ausschnitt aus Fig. 3,
- Fig. 5: einen Horizontalschnitt durch die Vorrichtung entlang der Linie V in Fig. 3,
- Fig. 6: einen Horizontalschnitt durch eine weitere Vorrichtung ähnlich Fig. 5,
- Fig. 7: eine teilweise geschnittene Seitenansicht einer Vorrichtung mit abnehmbarer Laufsohle,
- Fig. 8: den Grundriß eines evakuierbaren Kissens,
- Fig. 9: einen Horizontalschnitt durch eine weitere Vorrichtung in Höhe der Linie IX-IX der Fig. 3.

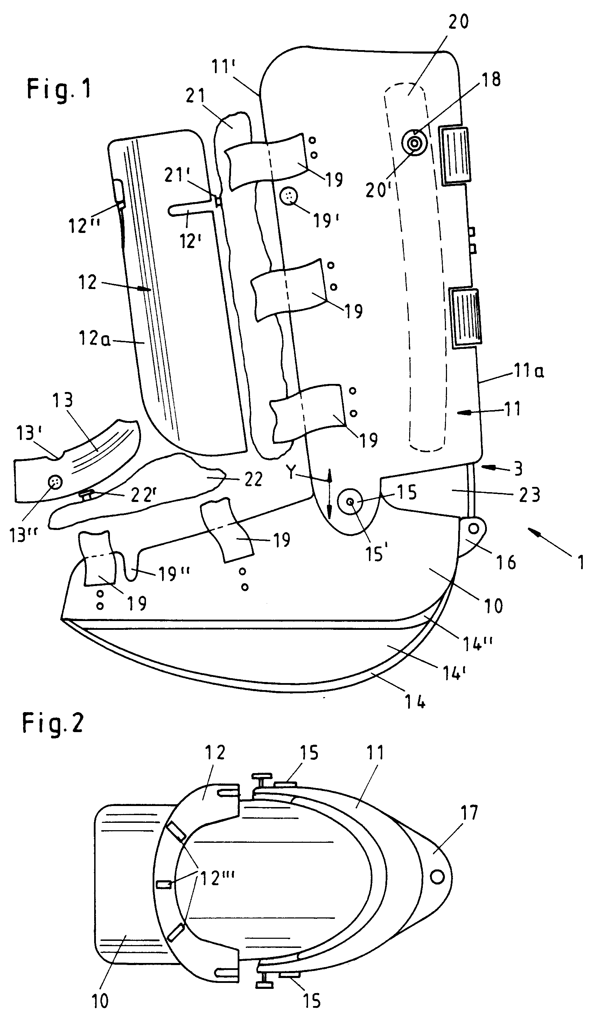

Eine Vorrichtung zur umschließenden Fixierung von Extremitäten ist in Fig. 1 als Schalenkörper 1 für den Fuß/Unterschenkel-Bereich eines Patienten dargestellt. Der Schalenkörper 1 besteht aus einem ersten Schalenteil 11, das einen Fußteil 10 und einen Schaftteil 11a umfaßt. Das Fußteil 10 und das Schaftteil 11a sind über seitliche Gelenke 15 schwenkbar miteinander verbunden. Die Gelenke bestehen bevorzugt aus Kunststoff, um den Durchtritt von Röntgenstrahlen zu gestatten.

Das Fußteil 10 ist schalenförmig nach oben offen ausgebildet, so daß eine Aufnahmewanne für einen Fuß geschaffen ist. An der Unterseite des Fußteils 10 ist eine Laufsohle 14 vorgesehen, deren äußere Gestalt an die Abrollbewegung des Fußes beim Gehen angepaßt ist. Zwischen der Laufsohle 14 und der Unterseite der Schale des Fußteils 10 sind Dämpfungsschichten 14', 14'' vorgesehen, wobei eine der Schichten eine stoßabsorbierende Schicht 14'' sein kann.

Im Fersenbereich des Fußteils 10 ist ein Lagerbock 16 für eine später erläuterte Verstelleinrichtung 3 angebracht.

Der schaftteil 11a besteht aus einem etwa halbschalenförmigen Körper, an dessen rückwärtigem Bereich ein Gehäuseabschnitt 17 zur Aufnahme der Verstelleinrichtung 3 vorgesehen ist. Im rückwärtigen Seitenbereich ist zumindest eine Öffnung 18 in der Wandung des Schaftteils 11 vorgesehen, die zum später erläuterten Durchtritt eines Ventils 20' dient. Die Öffnung 18 liegt vorzugsweise in der zur Körperaußenseite gerichteten Seitenwandung des Schaftteils 11a. Wahlweise kann aber in der gegenübergelegenen Seitenwandung des Schaftteils 11a eine weitere öffnung 18 vorgesehen sein, so daß die Vorrichtung an beiden Beinen verwendbar ist.

Im Bereich der vorderen Ränder des Schaftteils 11a ist an jedem Rand zumindest ein nach außen stehender Pilzzapfen 19' angeordnet. Die Elastizität der Seitenwandungen des Schaftteils 11a nimmt vom rückwärtigen Bereich des Schaftteils 11a zum vorderen, freien Rand 11' des Schaftteils zu.

Entlang eines jeden vorderen Randes des Schaftteils 11a sind mehrere Verspannelemente angeordnet, wobei jeweils zwei auf gleicher Höhe liegende Verspannelemente 19 als eine Verspanneinrichtung zusammenwirken. Die Verspannelemente 19 können beispielsweise durch Klettband gebildet sein, das am Schaftteil 11a befestigt, bspw. angenietet oder angeklebt, ist. Gleichartige Verspanneinrichtungen 19 sind auch an den freien Rändern des Fußteils 10 vorgesehen. Das Fußteil 10 weist jeweils an seinem oberen Rand im vorderen Bereich einen etwa rechtwinklig zum Rand verlaufenden Querschlitz 19'' auf.

Des weiteren besteht der Schalenkörper 1 aus einem zweiten Schalenteil 12, das eine Schaftabdeckschale 12a umfaßt und das ebenso wie der Schalenkörper 1 aus einem harten, geringfügig elastischen Material, beispielsweise einem Kunststoff besteht. Die Schaftabdeckschale 12a weist in ihrem oberen Bereich an den hinteren, freien Rändern ihrer Seitenwandungen jeweils einen Schlitz 12' auf, in den jeweils der Pilzzapfen 19' des Schaftteils 11 eingeführt wird, so daß auf diese Weise eine Führung für die Schaftabdeckschale 12a am Schaftteil 11 geschaffen ist. In ihrem vorderen Bereich besitzt die Schaftabdeckschale 12a in ihrer Wandung eine Öffnung 12'' zum Durchtritt eines Ventils 21'.

Eine Fußabdeckschale 13 ist ähnlich ausgebildet wie die schaftabdeckschale 12a und weist an ihrer Oberseite eine Wandungsöffnung 13' zum Durchtritt eines Ventils 22' auf. Seitlich besitzt die Fußabdeckschale 13 jeweils im Bereich ihres freien Randes einen Pilzzapfen 13'', der zusammen mit dem jeweiligen Schlitz 19'' im Fußteil 10 eine Führung für die Fußabdeckschale 13 bildet.

Die Innenseite der Schaftabdeckschale 12a sowie die Innenseite der Fußabdeckschale 13 können mit Rippen 12''', Stegen oder anderen nach innen weisenden Vorsprüngen versehen sein (Fig. 2). Diese Vorsprünge verhaken mit dem vorderen Kissen 21 und verhindern so ein Verrutschen des vorderen Kissens 21. Ähnliche Ausbildungen können auch an der Innenseite der Fußabdeckschale 13 für das Fußkissen 22 und/oder an der Innenseite des Schaftteiles 11a für das hintere Kissen 20 vorgesehen sein.

An der Innenseite des Schaftteils 11a ist ein hinteres Anpaßkissen 20 lose aufliegend oder an diesem - beispielsweise durch Klettband oder Kleber - befestigt angeordnet. Ein vorderes Anpaßkissen 21 ist auf der Innenseite der Schaftabdeckschale 12a vorgesehen, und ein gleichartiges Fußkissen 22 ist auf der Innenseite der Fußabdeckschale 13 vorgesehen.

Das hintere Kissen 20, das vordere Kissen 21 und das Fußkissen 22 bestehen aus einer vakuumdicht ausgebildeten und mit zumindest einem Evakuierungsventil 20', 21', 22' versehenen Blase, in deren Innenraum eine Vielzahl von relativ zueinander beweglichen Füllkörpern vorgesehen ist. Diese Füllkörper können beispielsweise Styroporkugeln (Polystyrolkugeln) sein.

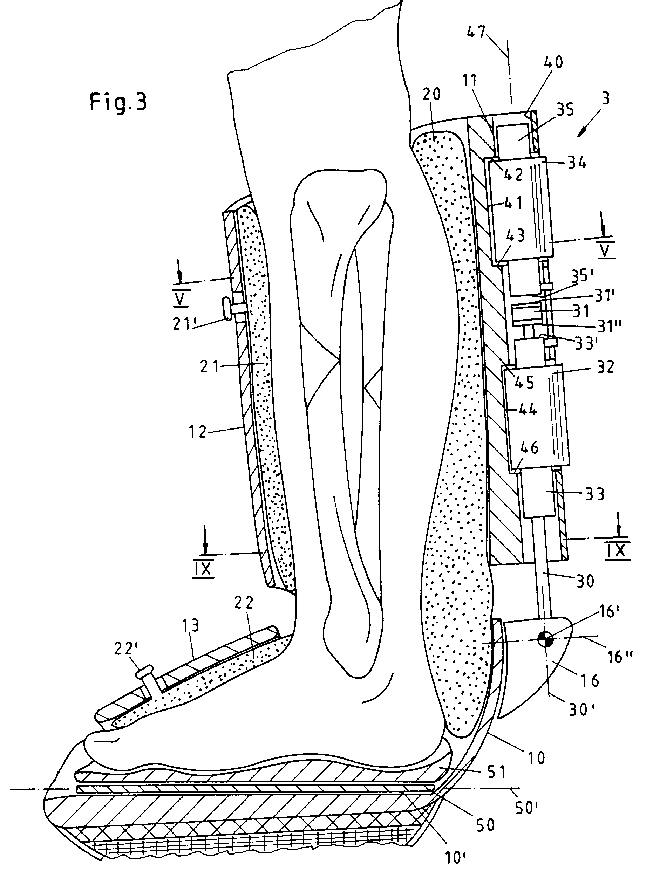

In Fig. 3 ist der Verstellmechanismus 3 zum Einstellen des Neigungswinkels zwischen Fußteil 10 und Schaftteil 11 sowie zum Einstellen des Winkelbereichs, in dem der Schaftteil 11a gegenüber dem Fußteil 10 schwenkbar ist, zu erkennen.

Im rückwärtigen Abschnitt des Schaftteils 11a ist in Schaftlängsrichtung ein im Querschnitt runder Kanal 40 vorgesehen. Im Kanal 40 sind voneinander beabstandet zwei Mutternaufnahmen 41, 44 ausgebildet, deren Durchmesser größer ist als der des Kanals und welche zur Rückseite des Schaftteils 11a hin offen sind. Den Übergang zwischen dem Kanal 40 und den Mutternaufnahmen 41, 44 bilden rechtwinklig zur Kanalachse 47 verlaufende Anschläge 42, 43; 45, 46.

In die Mutternaufnahmen 41, 44 sind rohrförmige, an ihrem Außenumfang mit einer Riffelung versehene Muttern eingesetzt. Der mit der Riffelung bzw. Rändelung versehene Umfang der Muttern ist dabei durch die hinteren Öffnungen der Mutternaufnahmen 41, 44 zugänglich.

In die obere Verstellmutter 34 ist eine Gewindestange 35 eingeschraubt, deren untere Stirnseite einen Anschlag 35' bildet. In die untere Verstellmutter 32 ist ein Gewinderohr 33 eingeschraubt, das an seiner Oberseite einen ringförmigen Anschlag 33' aufweist. Durch das Gewinderohr 33 ist ein beispielsweise von einer Metallstange gebildeter Anker 30 hindurchgesteckt, an dessen oberem Ende ein Anschlagkopf 31 fest angebracht ist. Der Anschlagkopf 31 ist dabei zwischen dem Anschlag 33' des Gewinderohrs 33 und dem Anschlag 35' der Gewindestange 35 gelegen. Die obere, zum Anschlag 35 weisende Stirnfläche des Anschlagkopfs 31 bildet einen oberen Anschlag 31' und die untere, zum Anschlag 33 weisende ringförmige Stirnfläche bildet einen unteren Anschlag 31''. Das andere Ende des Ankers 30 ist im Lagerbock 16 am Fußteil 10 gelenkig gelagert. Die Lagerachse 16' liegt dabei im rechten Winkel zur Achse 30' des Ankers 30 und verläuft im wesentlichen parallel zur Achse 15' der beiden Gelenke zwischen Schaftteil 11 und Fußteil 10.

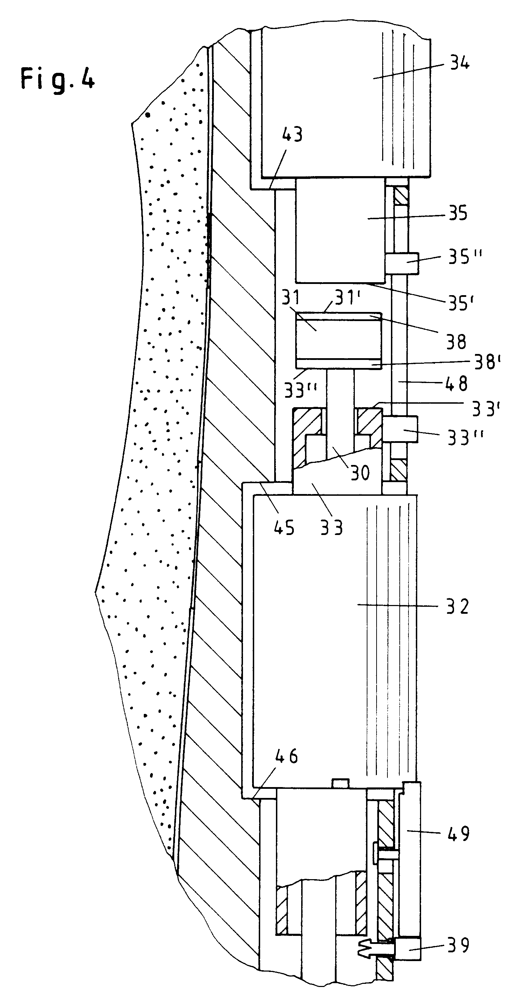

Wie in Fig. 4 zu erkennen ist, ist der Anker 30 von der Stirnwandung des Gewinderohrs 33 im Bereich des Anschlags 33' geführt, während über die verbleibende Länge des Gewinderohrs 33 dessen Innendurchmesser wesentlich größer ist als der Durchmesser des Ankers 30. Auf diese Weise wird ein Verklemmen des Ankers 30 im Gewinderohr 33 beim Verschwenken des Schaftteils 11 um den Fußteil 10 verhindert.

An der rückwärtigen Seite des Schaftteils 11a ist zwischen den Mutternaufnahmen 41, 44 ein Schlitz 48 vorgesehen, in den von innen Stifte 33'', 35'' ragen, welche im Bereich der jeweiligen Anschläge 33' bzw. 35' des Gewinderohrs 33 bzw. der Gewindestange 35 angebracht sind und diese an einer Drehung um ihre Längsachse innerhalb des Kanals 40 hindern.

Durch Verdrehen der oberen Verstellmutter 34 wird die Gewindestange 35 entlang des Kanals 40 verschoben. Auf die gleiche Weise wird durch Verdrehen der unteren Verstellmutter 32 das Gewinderohr 33 im Kanal 40 verschoben. Somit kann durch Verdrehen der oberen Verstellmutter 34 die Lage des Anschlags 35' der Gewindestange 35 und durch Verdrehen der unteren Verstellmutter 32 die Lage des Anschlags 33' des Gewinderohrs 33 innerhalb des Kanals 40 verändert werden. Der Anschlagkopf 31 ist aufgrund der festen Verbindung mit dem Fußteil bezüglich des Fußteils in Richtung der Kanalachse 47 im wesentlichen starr, so daß eine Schwenkbewegung des Schaftteils 11 um die Achse 15' nur innerhalb der Bereiche zwischen dem oberen Anschlag 31 ' des Anschlagkopfs 31 und dem Anschlag 35' der Gewindestange 35 sowie zwischen dem unteren Anschlag 31'' des Anschlagkopfs 31 und dem Anschlag 33' des Gewinderohrs 33 gestattet ist. Dabei begrenzt der Anschlag 35' der Gewindestange 35 die Schwenkbewegung des Schaftteils 11 nach hinten, während der Anschlag 33' des Gewinderohrs 33 den Schwenkbereich des Schaftes nach vorne begrenzt.

Auf diese Weise kann der zulässige Schwenkbereich des Schaftteiles 11a aus einer beliebig wählbaren Ruhestellung nach vorne unabhängig von dem Schwenkbereich aus dieser Ruhestellung nach hinten eingestellt werden. Darüber hinaus kann der Schaftteil 11a in jeder zwischen den einstellbaren Winkelpositionen wählbaren Winkelposition fixiert werden, wobei sowohl die Gewindestange 35 als auch das Gewinderohr 33 in Anlage mit dem Anschlagkopf 31 gebracht und verspannt werden. Bei diesem Verspannen wird die obere, ringförmige Stirnseite der Verstellmutter 34 gegen den oberen Anschlag 42 der oberen Mutternaufnahme 41 zur Anlage gebracht, während die untere ringförmige Stirnseite der unteren Verstellmutter 32 gegen den unteren Anschlag 46 der unteren Mutternaufnahme 44 in Anlage gerät. Aufgrund dieser Verspannung ist die gewählte Winkellage des Schaftteils 11 im Bezug auf das Fußteil 10 spielfrei festgelegt.

Um ein unbeabsichtigtes Verdrehen der Verstellmuttern 32 bzw. 34 zu verhindern, können Verdrehsicherungen für diese Muttern vorgesehen sein. Beispielsweise kann an der rückwärtigen Seite des Schaftes jeder Verstellmutter zugeordnet ein Schieber 49 vorgesehen sein, der zur Verriegelung der Verstellmutter 32 bzw. 34 mit dieser formschlüssig in Eingriff gerät. Eine Sicherung des jeweiligen Schiebers 49 gegen unbeabsichtigtes Lösen dieses Eingriffs kann zusätzlich, beispielsweise durch einen Stöpsel 39 erfolgen, der in eine bei verriegeltem Schieber 49 zugängliche Bohrung in der Rückseite des Schaftteils eingesetzt wird und dort durch Verrasten am Herausfallen gehindert ist.

Werden entlang des Schlitzes 48 Skalen an der Außenseite des Schaftteils 11 angebracht, so lassen sich der vordere und der hintere Grenzwinkel durch den in diesem Fall zusätzlich als Zeiger dienenden Stift 33'' bzw. 35'' ablesen. Der obere Anschlag 31' und der untere Anschlag 31'' des Anschlagkopfs 31 können mit einer elastischen und/oder stoßabsorbierenden Schicht 38, 38' versehen sein, so daß Stöße beim Verschwenken des Schaftteils 11a gegenüber dem Fußteil 10 - beispielsweise beim Gehen - gedämpft und ggf. absorbiert werden. Eine stoßdämpfende Funktion kann auch eine elastische Lagerung des Ankers 30 im Lagerbock 16 erfüllen.

In dem Fußteil 10 ist auf der unteren Innenfläche 10'' der Fußteilwanne eine Zwischensohle 50 angeordnet, die bezüglich des Fußteils 10 um eine parallel zur Fußlängsrichtung verlaufende Achse 50' geneigt werden kann. Darüber hinaus ist der Abstand der Zwischensohle 50 zur unteren Innenfläche 10'' des Fußteils 10 veränderbar. Auf diese Weise kann die Zwischensohle 50 sowohl in ihrer Höhe als auch in ihrer Neigung bezüglich der Innenfläche 10'' verstellt und in der gewählten Lage auch fixiert werden. Die Verstellung kann durch Einlegen von Elementen zwischen die Innenfläche 10'' und die Zwischensohle 50 oder durch einen nicht gezeigten Verstellmechanismus erfolgen.

Oberhalb der Zwischenoshle 50 ist ein Fußbett 51 angeordnet, das aus einem elastischen, stoßdämpfenden Material besteht.

Eine weitere Verstellmöglichkeit um eine parallel zur Fußlängsrichtung liegende Achse kann dadurch gegeben sein, daß die seitlichen Gelenke 15 unabhängig voneinander in ihrer Höhe (entlang des Pfeils Y in Fig. 1) verstellbar sind. Ist eine derartige Verstellung der Seitenneigung des Schaftteils 11a gegenüber dem Fußteil 10 vorgesehen, so sollte der Lagerbock 16 am Fußteil 10 ebenfalls um eine im wesentlichen parallel zur Fußlängsrichtung verlaufende Achse 16'' verschwenkbar sein, um Verspannungen innerhalb des Verstellmechanismus 3 zu verhindern.

Nachfolgend wird die Arbeits- und Wirkungsweise der Vorrichtung beschrieben. In die geöffnete und bereits mit dem hinteren Kissen 20 und dem Fußbett 51 versehene Vorrichtung werden Unterschenkel und Fuß eines Patienten eingelegt, wobei die Füllkörper im hinteren Kissen 20 vorher derart verteilt werden, daß die Form des hinteren Kissens 20 in etwa der Form des hinteren Unterschenkel- und Fußbereiches des Patienten entsprechen.

Daraufhin werden Unterschenkel und Fuß in der Vorrichtung ausgerichtet, wobei beispielsweise zum Einrichten eines Unterschenkelbruchs die elastischen Seitenwände des Schaftteiles 11 zur Seite gedrückt werden können. Außerdem kann die Gestalt des hinteren Kisens 20 durch Verschieben der Füllkörper bei Bedarf verändert werden. Vorteilhaft für den Zugang zum hinteren Kissen 20 ist dabei auch ein hinter den Gelenken 15 vorgesehener Freiraum 23 im Bereich der Achillessehne des Patienten.

Danach wird das vordere Kissen 21 auf den vorderen Unterschenkelbereich des Patienten aufgelegt, wobei auch hier die Füllkörper so verschoben werden, daß sich das Kissen der Kontur des Unterschenkels im vorderen Bereich anpaßt.

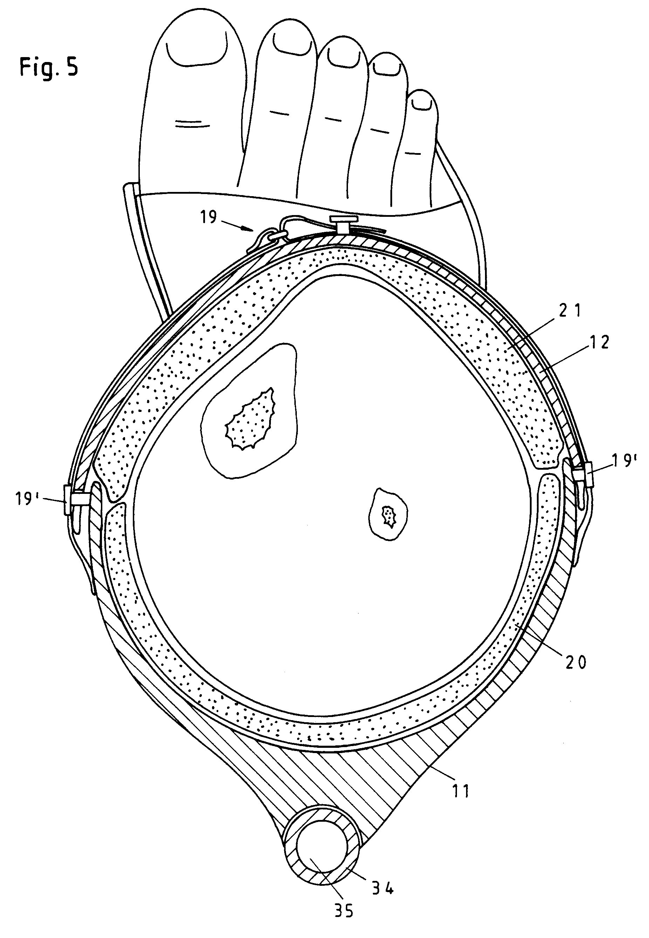

Daraufhin wird die Schaftabdeckschale 12, die vorderen Ränder des Schaftteiles 11 außen übergreifend (Fig. 5), auf das Schaftteil 11a aufgesetzt, wobei die Pilzzapfen 19' in die Schlitze 12' eingreifen. Die beispielsweise aus Klettband bestehenden Verspanneinrichtungen an der Vorderseite des Schaftteiles 11a werden über die Schaftabdeckschale geführt und dort derart verspannt, daß sie die Schaftabdeckschale gegen den in der Vorrichtung liegenden Unterschenkel drücken.

Auf die gleiche Weise wird dann das Fußkissen 22 auf die Oberseite des Fußes des Patienten gelegt und anschließend die Fußabdeckschale 13 in entsprechender Weise in die Schlitze 19'' des Fußteils 10 eingesetzt und mit den dort befindlichen Verspanneinrichtungen vorgespannt.

Anschließend wird die Luft durch die Ventile 20', 21', 22' aus den Kissen 20, 21 und 22 gesaugt, so daß die in dem jeweiligen Kissen befindlichen Füllkörper in einen engen Reibungskontakt geraten und auf diese Weise das jeweilige Kissen in der dem Unterschenkel bzw. dem Fuß des Patienten angepaßten Form im wesentlichen starr wird, ohne jedoch einen Druck auf den Patienten auszuüben.

Nach diesem Schritt können bei Bedarf die Verspanneinrichtungen nachgespannt werden. Die sich auf dem durch die Evakuierung harten Kissen, abstützende, steife und stabile Schale sorgt dafür, daß der Unterschenkel und der Fuß des Patienten dann fest in der Vorrichtung aufgenommen sind.

Da das Anlegen der Vorrichtung üblicherweise im Liegen des Patienten erfolgt, kann nach dem Aufstehen des Patienten im Stand ein Abstand zwischen Fußsohle und Fußbett 51 vorhanden sein, der ein gleichmäßiges Auftreten des Fußes verhindert. In diesem Fall kann durch eine Höhenverstellung der Zwischensohle 50 gegenüber der Innenseite 10' des Fußteils 10 das Fußbett 51 an die Fußsohle herangeführt werden. Die Zwischensohle 50 kann dabei über ihre gesamte Länge gleichmäßig oder auch nur bereichsweise höhenverstellt werden.

Zur optimalen Anpassung des Fußbetts an die Fußsohle des Patienten kann danach eine Canting-Verstellung erfolgen, wobei die Neigung der Zwischensohle 50 um die Achse 50' erfolgt. Für den Fall, daß eine extrem starke Canting-Korrektur notwendig ist, was beispielsweise bei Patienten mit ausgeprägten O-Beinen der Fall sein kann, läßt sich auch die Seitenneigung des Schaftteiles 11 gegenüber dem Fußteil durch Höhenverstellung eines oder beider Gelenke 15 verändern. Auf diese Weise sind sowohl der Fuß als auch der Unterschenkel fest und trotzdem ohne übermäßigen Druck auf den Körper des Patienten in der Vorrichtung aufgenommen.

Eine Bewegung im Fußgelenk des Patienten kann verhindert werden, indem mittels der beiden Verstellmuttern 32 und 34 der Neigungswinkel des Schaftteils 11a bezüglich des Fußteils 10 um die Achse 15' fest eingestellt wird. Eine Wahl dieses fest eingestellten Winkels kann in Abhängigkeit von der zu behandelnden Verletzung innerhalb des gesamten von der Verstelleinrichtung einstellbaren Schwenkwinkelbereichs erfolgen.

Bei Verletzungen, die eine Bewegung des Patienten im Fußgelenkbereich gestatten, kann der Schwenkwinkel um die Achse 15' herum durch Einstellen der Verstellmuttern 32 und 34 begrenzt werden, wobei die maximale Verschwenkung nach vorne und die maximale Verschwenkung nach hinten unabhängig voneinander einstellbar sind.

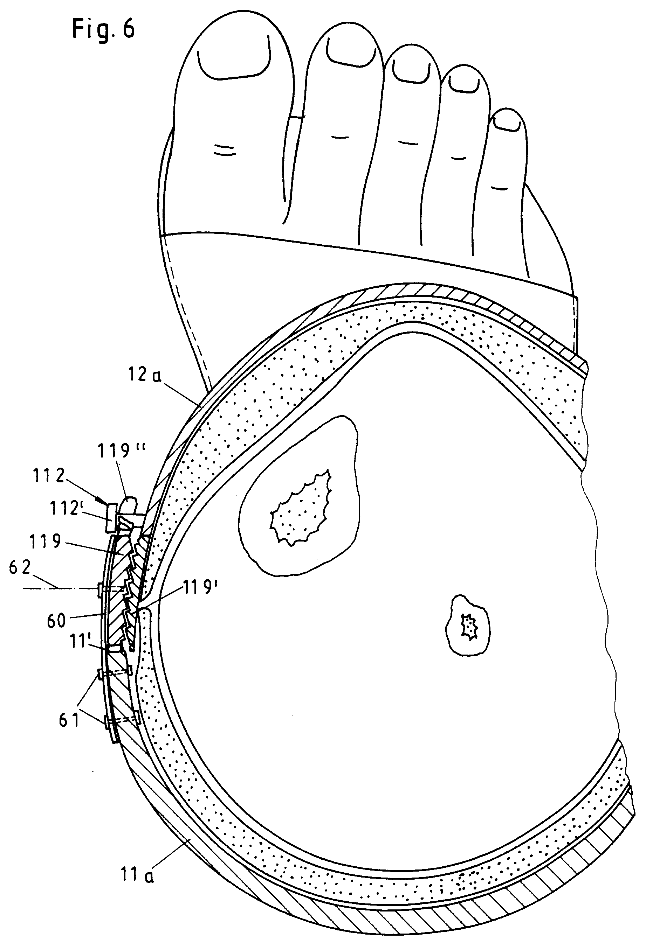

Fig. 6 zeigt eine weitere Ausführungsform der Vorrichtung bei der die Verschlußorgane aus einer Mehrzahl von Rastpositionen aufweisenden Rastvorrichtungen mit ineinander steckbaren Rastelementen 119, 119' bestehen. Bei dieser Ausführungsform übergreifen die freien Ränder des Schaftteils 11a die freien Ränder der Schaftabdeckschale 12a, so daß die freien Ränder der Schaftabdeckschale 12a zwischen den freien Rändern des Schaftteils 11a und des zugeordneten Kissens gelegen sind.

Im Bereich der freien Ränder des Schaftteils 11a sind Öffnungen 11' vorgesehen, die von einem Rastelement 119 federnd durchgriffen werden. Das Rastelement 119 weist dabei auf seiner Innenseite Rastzähne auf und ist an seiner Außenseite an einem auf herkömmliche Weise, beispielsweise mitels Nieten 61 am Schalenteil 11a angebrachten Federelement 60, vorzugsweise um eine Querachse 62 drehbar, befestigt. Die Feder 60 ist dabei derart vorgespannt, daß das Rastelement 119 durch die Öffnung 11' nach innen vorgespannt ist.

Am freien Randbereich der Schaftabdeckschale 12a sind starre Gegenrastelemente 119' vorgesehen, deren Rastzähne nach außen weisen, um mit den Rastzähnen eines auf gleicher Höhe gelegenen Rastelements 119 zusammenzuwirken. Dabei sorgt die Drehbarkeit des Rastelements 119 um die Querachse 62 dafür, daß sich die Lage des drehbaren Rastelements 119 an die Längserstreckung der Rastzähne des starren Elements 119' anpaßt.

Um ein für eine sichere Verrastung notwendiges Gegenlager zu schaffen, sind in der Nähe der Gegenrastelemente 119', vorzugsweise oberhalb und unterhalb der Gegenrastelemente 119', an der Schaftabdeckschale 12a Pilzzapfen 112 vorgesehen, die in auf gleicher höhe, ebenfalls vorzugsweise oberhalb und unterhalb des zugehörigen Rastelements 119 ausgebildete Schlitze 119'' im Schaftteil 11a eingreifen, und die diese Schlitze mit ihrem Pilzkopf 112' außen übergreifen.

Die federnd auslenkbaren Rastelemente 119 können vorzugsweise in der jeweiligen Verrastungsposition blockierbar sein, wobei sie insbesondere mitels eines separaten Betätigungsorgans absperrbar sind, so daß ein unautorisiertes Öffnen der Verrastung und damit Öffnen der Vorrichtung verhindert ist. Beispielsweise kann eine derartige Sicherung des auslenkbaren Rastelements 119 von einer mit radial hervorstehenden Nasen versehenen und am Federelement 60 angebrachten, drehbaren Scheibe gebildet sein, wobei die Nasen nach Art eines Bajonettverschlusses in am Schaftteil 11a vorgesehene Nuten eingreifen. Dabei kann die verdrehbare Scheibe so ausgestaltet sein, daß sie nur mittels eines speziellen Werkzeugs verdrehbar ist. Sind an dem Schaftteil außerdem Auflaufflächen für die Nasen vorhanden, so kann durch Verdrehen der Scheibe das federnde Rastelement 119 auch in eine abgehobene, geöffnete Stellung gebracht und dort festgelegt werden. In dieser Stellung ist dann ein problemloses Lösen der Rastvorrichtung durchführbar.

Derartige Rastvorrichtungen können selbstverständlich auch an der Außenseite der Vorrichtung sowie am Fußteil 10 und der Fußabdeckschale 13 vorgesehen sein. Außerdem ist eine gemischte Verwendung der vorbeschriebenen Rastvorrichtungen und der in Fig. 5 dargestellten Spannvorrichtungen möglich.

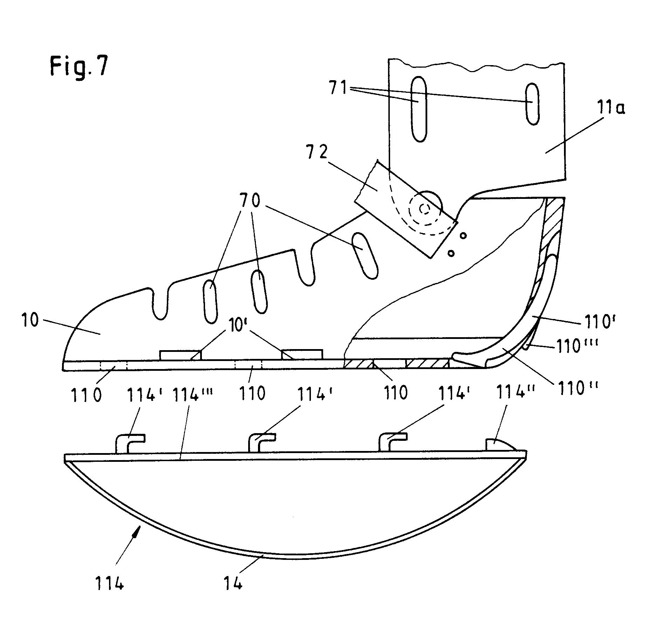

Fig. 7 zeigt eine weitere Ausführung der Vorrichtung nach der Erfindung, bei der die Laufsohle abnehmbar ist. Das Fußteil 10 besitzt dabei an seiner Unterseite Öffnungen 110, die vorzugsweise als sich in Längsrichtung des Fußteils erstreckende Langlöcher ausgebildet sind. Das abnehmbare Laufsohlenteil 114 ist mit einer nach unten gewölbten Laufsohle 14 versehen und besitzt auf seiner oberen, zum Fußteil 10 weisenden Seite eine starre Befestigungsplatte 114'''. An der Befestigungsplatte 114''' sind - jeder Öffnung 110 im Fußteil 10 zugeordnet - Verhakungselemente 114' ausgebildet, die aus der Befestigungsplatte nach oben hervorstehen und einen nach hinten gerichteten, hakenartigen Ansatz besitzen.

Am hinteren Ende der Befestigungsplatte 114''' ist ein mit einer nach vorne gerichteten Rastkante versehenes Gegenrastelement 114'' ausgebildet. Ein diesem Gegenrastelement 114'' zugeordneter, federnd gelagerter Rasthebel 110' ist im Fersenbereich des Fußteils 10 vorgesehen.

Zur Befestigung des Laufsohlenteils 114 am Fußteil 10 wird das Laufsohlenteil 114 von unten gegen das Fußteil 10 gedrückt, wobei die Verhakungselemente 114' in die Öffnungen 110 eindringen. Dann wird das Laufsohlenteil 114 nach hinten geschoben, wobei die hakenartigen Ansätze der Verhakungselemente 114' den hinteren Rand einer jeden Öffnung 110 übergreifen. Gleichzeitig wird beim Nach-Hinten-Schieben des Laufsohlenteils 114 der Rasthebel 110' von der schrägen hinteren Kante des Gegenrastelements 114'' ausgelenkt und schnappt in der hintersten Stellung des Laufsohlenteils 114 mit seiner Rastfläche vor die Rastfläche des Gegenrastelements 114''. auf diese Weise ist das Laufsohlenteil 114 sicher am Fußteil 10 befestigt.

Zum Lösen des Laufsohlenteils 114 wird der Rasthebel 110' an seinem hinten nach oben stehenden Betätigungshebel gegriffen und dieser nach unten gedrückt, wobei der Rasthebel 110' um seine Schwenkachse 110'' (in Fig. 7 im Uhrzeigersinn) geschwenkt wird und das Gegenrastelement 114'' freigibt. Ein ebenfalls am Rasthebel 110' vorgesehener Auswerferansatz 110''' drückt beim weiteren Schwenken des Hebels 110' gegen die hintere Kante der Befestigungsplatte 114''' des Laufsohlenteils 114 und schiebt dieses nach vorne, so daß die Verhakungselemente 114' aus den Öffnungen 110' des Fußteils 10 austreten können. Auf diese Weise ist ein einhändiges Lösen des Laufsohlenteils 114 vom Fußteil 10 ohne zusätzliches Werkzeug möglich. Vorteilhaft ist ein derartiges abnehmbares Laufsohlenteil bezüglich der Hygiene, denn es kann vom Patienten abgenommen werden, wenn er mit der Vorrichtung ins Bett geht. Gleichzeitig kann bei mehrmaliger Verwendung der Vorrichtung das als Verschleißteil ausgebildete Laufsohlenteil einfach und kostengünstig ersetzt werden.

Ein weiterer Vorteil eines derartigen abnehmbaren Laufsohlenteils ist darin zu sehen, daß die Vorrichtung ohne Laufsohlenteil als Ersatz für einen "Liegegips" verwendbar ist, wobei für den Patienten erkennbar ist, daß er mit der Vorrichtung ohne Laufsohle noch nicht gehen darf. Erst wenn beispielsweise der behandelnde Arzt die Laufsohle an den Patienten aushändigt, ist dies für den Patienten das deutliche Zeichen dafür, daß er nunmehr gehen und das Bein wieder belasten darf.

In Fig. 7 sind weiterhin Belüftungsöffnungen 70 bzw. 71 im Fußteil 10 und im Schaftteil 11a ausgebildet. Diese Belüftungsöffnungen 70, 71 sind Teile eines später beschriebenen Belüftungssystems für die in der Vorrichtung aufgenommenen Körperteile.

Weiterhin sind im Boden des Fußteils 10 im Bereich der Fußaufstandsfläche nach außen mündende Drainagekanäle 10' zur Ableitung von sich im Fußteil ansammelnder Flüssigkeit vorgesehen. Die im Fußteil angeordnete Zwischensohle 50 sowie das Fußbett 51 sind in diesem Fall mit (nicht gezeigten) Durchlässen versehen, die in die Drainagekanäle 10' münden und das Abfließen von vom Fuß abgegebenem Schweiß oder in die Vorrichtung eindringendem Wasser gestatten.

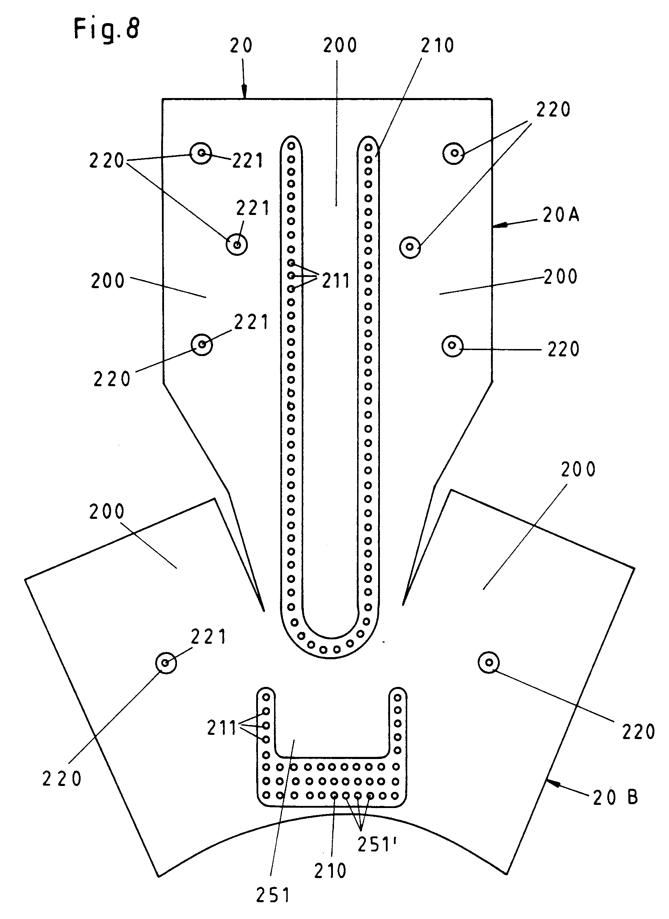

In Fig. 8 ist ein flächiges, evakuierbares Kissen gezeigt, wobei das Ventil auf der nicht einsehbaren Rückseite liegt. Das Kissen besitzt eine Mehrzahl von untereinander verbundenen und mit Füllkörpern gefüllten vakuumdichten Bereichen 200, zwischen denen Stege 210 und Inseln 220 ausgebildet sind. Die Stege 210 und die Inseln 220 sind mit Luftdurchtrittsöffnungen 211, 221 versehen. Die Stege 210 verlaufen dabei vorzugsweise in Richtung der Längserstreckung des Kissens 20.

Im evakuierten Zustand der Kissen bilden die Stege Be- und Entlüftungskanäle. Weitere Be- und Entlüftungskanäle werden durch beim Evakuieren des Kissens entstehende negative Falten im Kissen gebildet,so daß sich auch zwischen den Inseln 220 und den Stegen 210 Kanäle ausbilden. Durch diese Kanäle und die Luftdurchtrittsöffnungen 211, 221 kann ein Luftaustausch zwischen der Oberfläche des umschlossenen Körperteils und der Außenseite des dieses Körperteil umschließenden Kissens stattfinden. Auch die durch die Füllkörper bedingte unebene Oberfläche des Kissens im evakuierten Zustand sorgt für die Bildung kleinster Be- und Entlüftungskanäle.

Das Kissen 20 ist in den Schalenteilen derart angeordnet, daß zuminest die von den Stegen 210 gebildeten Kanäle vorzugsweise aber auch die Inseln 220 im Bereich der Luftdurchlässe 70, 71 des zugehörigen Schalenteils 11, 12 gelegen sind. Dadurch wird der Luftaustausch zwischen den umschlossenen Körperteilen und der Außenseite der Vorrichtung gewährleistet.

Die Schaftabdeckschale 12a, die Fußabdeckschale 13 und die zugeordneten Kissen 21, 22 können anlog ausgestaltet sein. Das Kissen 20 ist zumindest auf der dem zu umschließenden Körperteil zugewandten Seite mit einer zum Feuchtigkeitsabtransport dienenden Trockenschicht und einer sich daran anschließenden feuchtigkeitsaufnehmenden Speicherschicht versehen. Auf diese Weise wird ein Klimasystem geschaffen, daß eine zuverlässige Belüftung der umschlossenen Körperteile gewährleistet und gleichzeitig auch für einen Feuchtigkeitsabtransport von der Haut der umschlossenen Körperteile sorgt. Die Füllung des Kissens aus Polystyrol sorgt dabei gleichzeitig für eine Wärmeisolierung der umschlossenen Körperteile, was beispielsweise insbesondere im Winter vorteilhaft ist.

Das in der Figur 8 dargestellte Kissen 20 besteht aus einem oberen Schaftabschnitt 20 A und einem unteren Fußschnitt 20 B. Auf diese Weise kann mit einem einzigen evakuierbaren Kissen sowohl der Unterschenkel als auch der Fuß eines Patienten umschlossen werden. Im mittleren Bereich des Fußabschnitts 20 B ist ein vakuumdichter Bereich als Fußbett 251 ausgebildet, dessen Kontur sich beim Evakuieren des Kissens 20 an das Fußgewölbe des Patienten anpaßt und auf diese Weise gleichzeitig den Fuß stützt. Unter dem Fuß sind ebenfalls Luftdurchtrittsöffnungen 251' ausgebildet, die in die Drainagekanäle 10' münden und somit für eine Flüssigkeitsabfuhr aus dem Bereich unterhalb des Fußes sorgen.

Im Bereich des Übergangs vom Fußteil 10 zum Schaftteil 11a kann insbesondere angrenzend an das Schwenkgelenk 15 ein Spannelement, insbesondere ein Spannbügel 72, am Fußteil 10 oder am Schaftteil 11a angelenkt sein, um in diesem Bereich sowohl eine Querverspannung als auch ein Andrücken der zugehörigen Schaftabdeckschale an den Fuß zu erzielen (Fig. 7). Dieser Spannbügel 72 wirkt dem durch die Elastizität des Materials der Schalenteile 11, 12 bedingten seitlichen Aufbiegen in diesem Bereich entgegen und drückt somit auch in diesem Bereich das Schaftteil und das Fußteil seitlich gegen das zu umschließende Körperteil.

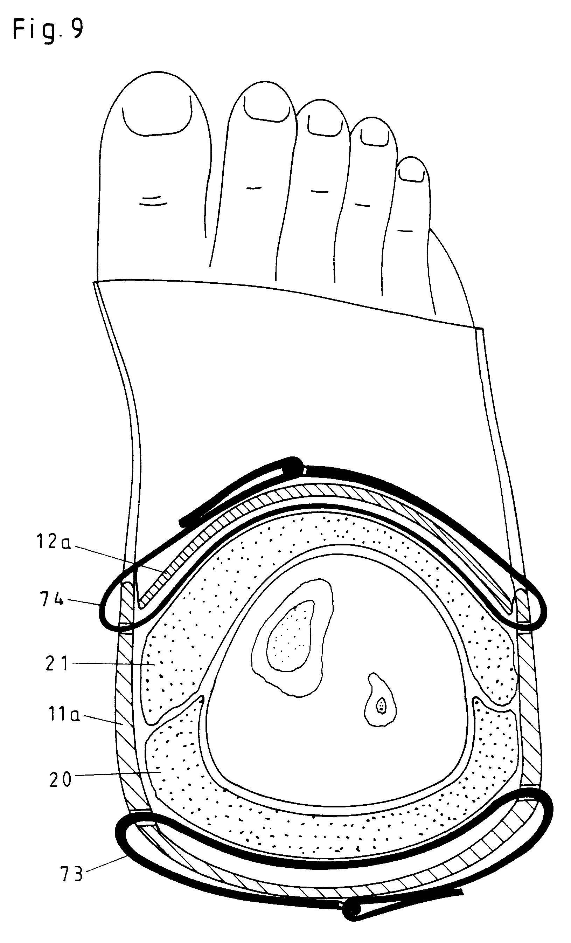

In Fig. 9 ist eine weitere besondere Ausführungsform der Vorrichtung nach der Erfindung dargestellt, wobei der Schaftbereich in Höhe der in Fig. 3 gezeigten Linie IX-IX geschnitten ist. Im hinteren Bereich des Schaftteils 11a ist ein Spannband 73 vorgesehen, daß das Schaftteil 11 von links nach rechts durchdringt, wobei das Spannband 73 durch seitliche Öffnungen des Schaftteils 22a geführt ist. Auf der rückwärtigen Außenseite des Schaftteils 11a ist das Spannband 73 auf herkömmliche Weise durch eine Schlaufe geführt. Auf der Innenseite des Schaftteils ist das Spannband zwischen dem Schaftteil 11 und dem Kissen 20 gelegen.

Wird nun das Spannband 73 gespannt, so wird der innerhalb des Schaftteils 11a gelegene Bereich des Spannbandes 73 gestreckt,wodurch ein in Fußrichtung nach vorne gerichteter Druck auf das Kissen 20 ausgeübt wird. Dieser Druck bewirkt eine Vorverlagerung des Fußes und insbesondere des Sprunggelenkes und bewirkt so eine Fixierung des eingeschlossenen Körperteils in dieser vorderen Lage.

Alternativ dazu kann ein Spannband 74 im vorderen Bereich des Schaftteils 11a vorgesehen sein, wobei das Spannband 74 seitliche Öffnungen im Bereich des freien Randes des Schaftteils 11a durchdringt und mit seinem inneren Abschnitt zwischen dem vorderen Kissen 21 und er Schaftabdeckschale 12a zu liegen kommt. Die äußeren Abschnitte des Spannbandes 74 werden außen um die Schaftabdeckschale 12a herumgeführt und dort miteinander verspannt.

Ein Festspannen des vorne angeordneten Spannbandes 74 bewirkt dabei einen Druck von vorne auf das vordere Kissen 21 und drückt so den Fuß und insbesondere das Sprunggelenk nach hinten gegen das Schaftteil 11a bzw. den Fersenbereich des Fußteils 10 und fixiert ihn in dieser Lage.

In bestimmten späteren Heilungsphasen kann die Evakuierung der Kisen 20, 21, 22 durch Aufbringen eines leichten Überdrucks ersetzt werden, wodurch sich eine erwünschte Massagewirkung mit Unterstützung durch die im Kissen enthaltenen Teilchen ergibt.

## Patentansprüche

1. Vorrichtung zur umschließenden Fixierung von Extremitäten und Extremitätenbereichen, insbesondere zur Behandlung von Extremitätenfrakturen im Bereich des Unterschenkels, bestehend aus einer die Extremität umschließenden, zu einer Hülse verformbaren, vakuumdicht ausgebildeten, evakuierbaren und mit einem Ventil (20') versehenen Manschettenkissen (20), in dessen zwischen den beiden Kissenwänden gelegenem Innenraum eine Vielzahl von losen Füllkörperteilchen vorgesehen ist und das durch Evakuierung zu einem in sich steifen, stabilen Gebilde verfestigbar ist, und aus einer äußeren, das unter Vakuum stehende Manschettenkissen (20) stabilisierenden Hülse (1), die in etwa die Form des Unterschenkels aufweist und schalenartig ausgebildet ist,
dadurch gekennzeichnet,
daß zumindest zwei gegeneinander verspannbare Schalenteile (11, 12) vorgesehen sind, die die zu umschließende Extremität umgeben, wobei das erste Schalenteil (11) einen Fußteil (10) und einen mit diesem gelenkig verbundenen Schaftteil (11a) und das zweite Schalenteil (12) eine Schaftabdeckschale (12a) und eine Fußabdeckschale (13) bilden, daß die beiden Schalenteile (11, 12) jeweils durch verstellbare Verschlußorgane (19, 19', 19''; 119, 119') randseitig überlappend verbindbar sind und daß zwischen den Schalenteilen (11, 12) und der Extremität ein oder mehrere verformbare, vakuumdicht ausgebildete und mit zumindest einem Ventil (20', 21', 22') versehene, evakuierbare Kissen (20, 21, 22) vorgesehen sind, wobei zwischen vakuumdichten Bereichen (200) der Kissen (20; 21; 22) im wesentlichen in Richtung der Längserstreckung der Kissen (20, 21, 22) verlaufende Stege (210) und/oder Inseln (220) ausgebildet sind, die mit Luftdurchtrittsöffnungen (211; 221) versehen sind und im evakuierten Zustand der Kissen zumindest zum Teil Be- und Entlüftungskanäle bilden.

2. Vorrichtung nach Anspruch 1, dadurch gekennzeichnet, daß die Verschlußorgane zumindest zum Teil aus eine Mehrzahl von Rastpositionen aufweisenden Rastvorrichtungen mit ineinander steckbaren Rastelementen (119, 119') bestehen.

3. Vorrichtung nach einem der Ansprüche 1 und 2,
dadurch gekennzeichnet,
daß die Rastvorrichtungen an einem Schalenteil (11) angeordnete, federnd auslenkbare Rastelemente (119) und am zugehörigen anderen Schalenteil (12) vorgesehene starre Gegenrastelemente (119') umfassen, und daß die federnd auslenkbaren Rastelemente (119) in der jeweiligen Verrastungsposition blockier- und vorzugsweise mittels eines separaten Betätigungsorgans absperrbar sind.

4. Vorrichtung nach einem der Ansprüche 1 bis 3,
dadurch gekennzeichnet,
daß zwischen den miteinander zu verbindenden Schalenteilen Formchluß-Führungen ausgebildet sind.

5. Vorrichtung nach einem der Ansprüche 1 bis 3,
dadurch gekennzeichnet,
daß mit der Unterseite des Fußteils (10) ein Laufsohlenteil (114) form- und kraftschlüssig kuppelbar ist.

6. Vorrichtung nach Anspruch 5,
dadurch gekennzeichnet,
daß das Laufsohlenteil (114) an seiner zum Fußteil (10) gewandten Seite starre Verhakungselemente (114'), die in Öffnungen (110) des Fußteils (10) einführ- und verhakbar sind, sowie ein Gegenrastelement (114'') aufweist, das mit einem im Fußteil (10), vorzugsweise in dessen Fersenbereich federnd gelagertem Rasthebel (110') zusammenwirkt.

7. Vorrichtung nach Anspruch 6,
dadurch gekennzeichnet,
daß der Rasthebel (110') mit einem Auswerfansatz (110'') für das Sohlenelement (114) versehen ist.

8. Vorrichtung nach einemder Ansprüche 1 bis 7,
dadurch gekennzeichnet,
daß die Schalenteile (10, 11, 12, 13) mit ihren einzelnen Teilen mit einer Mehrzahl von über die Schalenfläche verteilten Durchlässen (70, 71) vorgebbarer Größe und Abmessung versehen sind und daß insbesondere derartige Durchlässe im Bereich der von den Kissenstegen gebildeten Be- und Entlüftungskanäle vorgesehen sind.

9. Vorrichtung nach einem der Ansprüche 1 bis 8,
dadurch gekennzeichnet,
daß im Boden des Fußteils (10) im Bereich der Fußaufstandsfläche nach außen mündende Drainagekanäle (10') zur Ableitung von sich im Fußteil ansammelnder Füssigkeit vorgesehen sind.

10. Vorrichtung nach einem der Ansprüche 1 bis 9,
dadurch gekennzeichnet,
daß die Kissen (20, 21, 22) zumindest auf der dem zu umschließenden Körperteil (6) zugewandten Seite mit einer zum Feuchtigkeitsabtransport dienenden Trockenschicht und einer sich daran anschließenden feuchtigkeitsaufnehmenden Speicherschicht versehen sind.

11. Vorrichtung nach einem der Ansprüche 1 bis 10,
dadurch gekennzeichnet,
daß auf der Innenseite des Fußteils (10) ein Fußbett (251) vorgesehen ist, das als Bereich eines unter dem Fuß angeordneten evakuierbaren und Füllkörper enthaltenden Kissens ausgebildet ist.

12. Vorrichtung nach Anspruch 11,
dadurch gekennzeichnet,
daß im unter dem Fuß angeordneten Kissen Luftdurchtrittsöffnungen (251') vorgesehen sind, die in die Drainagekanäle (10') des Fußteils (10) münden und durch die unter dem Fuß angesammelte Flüssigkeit abgeführt wird.

13. Vorrichtung nach einem der Ansprüche 1 bis 12,
dadurch gekennzeichnet,
daß Fußteil (10) und Schaftteil (11a) über seitliche Gelenke (15) miteinander verbunden sind und daß im Fersenbereich eine am Fußteil (10) und am Schaftteil (11a) gelagerte Verstelleinrichtung (3) vorgesehen ist, mittels derer der Neigungswinkel des Schaftteils (11a) gegenüber dem Fußteil (10) einstellbar ist.

14. Vorrichtung nach Anspruch 13,
dadurch gekennzeichnet,
daß die Verstelleinrichtung (3) einen am Fußteil (10) gelenkig gelagerten Anker (30) aufweist, der ein in einer am Schaftteil (11) verdrehbar gelagerten Verstellmutter (32) eingeschraubtes und im Schaftteil (11a) unverdrehbar aber axial verschieblich aufgenommenes Gewinderohr (33) durchgreift und an seinem freien Ende mit einem Anschlagkopf (31) versehen ist, der mit einem vom zugeordneten Ende des Gewinderohrs (33) gebildeten Anschlag (33') zusammenwirkt.

15. Vorrichtung nach Anspruch 14,
dadurch gekennzeichnet,
daß am Schaftteil (11) eine zweite Verstellmutter (34) oberhalb der erten Verstellmutter (32) verdrehbar gelagert ist und daß eine Gewindestange (35) in die zweite Verstellmutter (34) eingeschraubt und im Schaftteil (11) unverdrehbar aber axial verschieblich geführt ist, wobei die untere, dem Anschlagkopf (31) zugewandte Stirnfläche der Gewindestange (35) einen weiteren Anschlag (35') für den Anschlagkopf (31) des Ankers (30) bildet, so daß oberhalb und unterhalb des Anschlagkopfes (31) je ein individuell einstellbarer Anschlag vorgesehen ist.

16. Vorrichtung nach Anspruch 14 oder 15,
dadurch gekennzeichnet,
daß zur Vermeidung einer unerwünschten Manipulation eine insbesondere absperrbare Verdrehsicherung (49, 39) für die Verstellmutter (32 und/oder 34) vorgesehen ist.

17. Vorrichtung nach einem der Ansprüche 1 bis 16,
dadurch gekennzeichnet,
daß die Seitenneigung des Schaftteils (11a) gegenüber dem Fußteil (10), vorzugsweise durch höhenverstellbare Gelenke (15), quer zur Gelenkachse (15') verstellbar ist.

18. Vorrichtung nach einem der Ansprüche 1 bis 17,
dadurch gekennzeichnet,
daß an der Innenseite des Schaftteiles (11a) und/oder an der Innenseite der Schaftabdeckschale (12a) und/oder an der Innenseite der Fußabdeckschale (13) nach innen weisende Vorsprünge (12''') zur Positionsfixierung der Kissen vorgesehen sind.

19. Vorrichtung nach einem der Ansprüche 1 bis 18,
dadurch gekennzeichnet,
daß am Fußteil (10) oder am Schaftteil (11a) im Bereich des Sprunggelenks eine Spannvorrichtung, insbesondere ein Spannband (73; 74), vorgesehen ist, mittels derer der Fuß und insbesondere das Sprunggelenk nach vorne oder nach hinten drückbar ist.

20. Vorrichtung nach einem der Ansprüche 1 bis 19,
dadurch gekennzeichnet,
daß im Bereich des Übergangs vom Fußteil (10) zum Schaftteil (11a) insbesondere angrenzend an das Schwenkgelenk, ein Spannelement, insbesondere ein Spannbügel (72), am Fußteil (10) oder am Schaftteil (11a) angelenkt ist, um in diesem Bereich sowohl eine Querverspannung als auch ein Andrücken der zugehörigen Schaftabdeckschale an den Fuß zu erzielen.

## Claims

1. Device for the comprehensive immobilization of limb ends and limb end regions, more particularly for extremity fractures within the area of the lower leg, which is comprised of a sleeve-like padding (20) ensheathing the extremity that is deformable into a collar-like sheath and constructed so as to be vacuum-tight, whose contents can be evacuated and provided with a valve (20'), in whose interior located between the two padding walls, a great number of loose filling material particles are provided and which, by means of an evacuation, can be consolidated so as to form an inherently rigid, stable structure, and of an external sheath (1) stabilizing the sleeve-like padding (20) acted upon by a vacuum which possesses the approximate configuration of the lower leg and is constructed in a shell-like fashion,
**characterized in that**
at least two shell portions (11,12) which can be tightened against each other are provided which enclose the extremity to be ensheathed, in which case the first shell portion (11) forms a foot portion (10) and a leg portion (11a) hingedly connected with the same and the second shell portion (12) forms a leg covering shell (12a) and a foot covering portion shell (13), in that the two shell portions (11,12) are in each case connectable so as to overlap on the edge side by means of adjustable closing elements (19,19',19'', 119,119') and in that, between the shell portions (11,12) and the extremity, one or several deformable paddings (20,21,22) constructed so as to be vacuum-tight, whose contents can be evacuated are provided fitted with at least one valve (20',21',22'), wherein, between vacuum-tight areas (200) of the paddings (20;21;22), webs (210) and/or islands (220) proceeding substantially in the direction of the longitudinal extension of the paddings (20,21,22) are constructed, which are provided with air passage apertures (211;221) and, in the evacuated state of the paddings, form at least in part ventilation and venting ducts.

2. Device according to Claim 1,
**characterized in that**
the closing elements are comprised at least in part of locking means possessing a plurality of locking positions with locking elements (119,119') which are insertable into each other.

3. Device according to either Claim 1 or 2,
**characterized in that**
the locking means comprise springably deflectable locking elements (119) disposed on a shell portion (11) and rigid counter locking elements (119') provided on the associated other shell portion (12), and in that the springably deflectable locking elements (119) are, in the respective locking position, blockable and, preferably by means of a separating actuating element, lockable as well.

4. Device according to any of Claims 1 to 3,
**characterized in that**
form-fitting guiding means are constructed between the shell portions to be interconnected.

5. Device according to any of Claims 1 to 3,
**characterized in that**
an outsole portion (114) can be coupled form-fittingly and rigidly connected to the underside of the foot portion.

6. Device according to Claim 5,
**characterized in that,**
the outsole portion (114), on its side facing the foot portion (10), possesses rigid hooking elements (114') which are introduceable and hookable into openings (110) of the foot portion (10) as well as a counterlocking element which interacts with a locking lever (110') in the foot portion (10), by preference within the heel area of the same.

7. Device according to Claim 6,
**characterized in that**
the locking lever (110') is provided with an ejection shoulder (110'') for the outsole element (114).

8. Device according to any of Claims 1 to 7,
**characterized in that**
the shell portions (10,11,12,13) with their individual components are provided with a plurality of passages (70, 71) distributed over the shell area of predeterminable size and dimension and in that, in particular, such passages are provided within the area of the ventilating and venting ducts formed by the padding webs.

9. Device according to any of Claims 1 to 8,
**characterized in that,**
in the bottom of the foot portion (10), within the area of the foot standing surface, outwardly terminating draining ducts (10') are provided for the removal of liquid accumulating in the foot portion.

10. Device according to any of the Claims 1 to 9,
**characterized in that,**
the paddings (20,21,22), at least on the side facing the part of the body (6) to be ensheathed, are provided with a dry layer serving to evacuate moisture and a moisture-absorbing storage layer following the same.

11. Device according to any of Claims 1 to 10,
**characterized in that,**
on the inside of the foot portion (10), a foot bedding (251) is provided which is constructed in the form of an area of a padding disposed underneath the foot, whose contents can be evacuated and which contains particles of filling material.

12. Device according to Claim 11,
**characterized in that,**
in the padding disposed underneath the foot, air passage apertures (251') are provided which lead into the draining ducts (10') of the foot portion (10) and through which the liquid accumulated underneath the foot is evacuated.

13. Device according to any of Claims 1 to 12,
**characterized in that**
the foot portion (10) and the leg portion (11a) are inter-connected by means of lateral hinges (15) and in that, within the heel area, an adjustment means (3) supported on the foot portion (10) and on the leg portion (11a) is provided with the aid of which the angle of inclination of the leg portion (11a) is adjustable in relation to the foot portion (10).

14. Device according to Claim 13,
**characterized in that**
the adjusting means (3) posssesses an anchor-like connection piece (30) hingedly supported on the foot portion (10) which reaches through a threaded tube (33) screwed into an adjusting nut (32) rotatably supported on the leg portion (11a) so as to be non-rotatable but axially displaceable and which, on its free end, is provided with a limit stop head (31), which interacts with a limit stop (33') formed by the allocated end of the threaded tube (33).

15. Device according to Claim 14,
**characterized in that,**
on the leg portion (11a), a second adjusting nut (34) is rotatably supported above the first adjusting nut (32) and in that a threaded rod (35) is screwed into the second adjusting nut (34) and guided non-rotatably, but axially displaceable in thea leg portion (11), while the lower front area of the threaded rod (35) facing the limit stop head (31) forms a further limit stop (35') for the limit stop head (31) of the anchor-like connection piece (30) so that, above and below the limit stop head (31), one individually adhustable limit stop each is provided.

16. Device according to either Claim 14 or 15,
**characterized in that,**
for the prevention of undesirable manipulation, a specially lockable protection againt rotation (49,39) for the adjusting nut (32 and/or 34) is provided.

17. Device according to any of Claims 1 to 16,
**characterized in that**
the lateral inclination of the leg portion (11a) in relation to the foot portion (10) is adjustable transversally to the hinge axis (15'), preferably by means of vertically adjustable hinges (15).

18. Device according to any of Claims 1 to 17,
**characterized in that,**
on the inside of the leg portion (11a) and/or on the inside of the leg covering shell (12a) and/or on the inside of the foot covering shell ( (13), inwardly pointing projections (12''') are provided for the positional fixation of the paddings.

19. Device according to any of Claims 1 to 18,
**characterized in that,**
on the foot port ion (10) or on the leg portion (11a) within the area of the ankle, a tightening means, more particularly a tensioning band (73;74) is provided, with the aid of which the foot and, in particular, the ankle can be pressed forward or backward.

20. Device according to any of Claims 1 to 19,
**characterized in that,**
within the area of transition from the foot portion (10) to the leg portion (11a), more particularly adjacent to the swivel hinge, a tightening element, especially a clamp (72), is hinged onto the foot portion (10) or onto the leg portion (11a) so as to achieve, in this area, both a transversal tension as well as a pressing of the associated leg covering shell against the foot.

## Revendications

1. Dispositif pour la fixation enveloppante d'extrémités ou de zones d'extrémités, en particulier pour le traitement des fractures des extrémités dans la zone de la jambe, constitué par un coussin à manchette (20) enveloppant l'extrémité, déformable en une gaine, configuré étanche au vide, dans lequel le vide peut être fait et pourvu d'une valve (20'), coussin dans l'espace intérieur, situé entre les deux parois de coussin, duquel une multitude de particules de corps de remplissage mobiles est prévue et qui peut être consolidé en un corps rigide stable en faisant le vide, et par une gaine extérieure (1), qui stabilise le coussin à manchette (20) sous vide, qui présente à peu près la forme de la jambe et qui est configurée de type coque,
**caractérisé en ce**
qu'au moins deux parties de coque (11, 12) pouvant être serrées l'une contre l'autre sont prévues qui entourent l'extrémité à envelopper, la première partie de coque (11) formant une partie pied (10) et une partie tige (11a) reliée à celle-ci de manière articulée et la seconde partie de coque (12) formant une coque de recouvrement de la tige (12a) et une coque de recouvrement du pied (13), que les deux parties de coque (11, 12) peuvent être reliées en se chevauchant côté bord respectivement par des organes de fermeture réglables (19, 19', 19'' ; 119, 119') et qu'un ou plusieurs coussins (20, 21, 22) déformables, dans lesquels on peut faire le vide, configurés étanches au vide et pourvus d'au moins une valve (20', 21', 22') sont prévus entre les parties de coque (11, 12) et l'extrémité, des barrettes (210) et/ou des îles (220) essentiellement dans le sens de l'extension en longueur des coussins (20, 21, 22) étant configurées entre les zones étanches au vide (200) des coussins (20 ; 21 ; 22), barrettes qui sont pourvues d'ouvertures de passage de l'air (211 ; 221) et qui, les coussins étant à l'état où on a fait le vide, forment au moins partiellement des canaux d'aération et de purge d'air.

2. Dispositif selon la revendication 1, **caractérisé en ce** que les organes de fermeture consistent au moins partiellement en une multitude de dispositifs d'enclenchement présentant des positions d'enclenchement avec des éléments d'enclenchement (119, 119') pouvant être enfichés les uns dans les autres.

3. Dispositif selon l'une des revendications 1 et 2,
**caractérisé en ce**
que les dispositifs d'enclenchement comprennent des éléments d'enclenchement (119) placés sur une partie de coque (11), pouvant dévier élastiquement, et des éléments d'enclenchement antagonistes (119') rigides prévus sur l'autre partie de coque correspondante (12) et que les éléments d'enclenchement (119) pouvant dévier élastiquement peuvent être bloqués dans la position d'enclenchement respective et de préférence arrêtés au moyen d'un organe d'actionnement séparé.

4. Dispositif selon l'une des revendications 1 à 3,
**caractérisé en ce**
que des guidages à engagement positif sont configurés entre les parties de coque devant être reliées l'une à l'autre.

5. Dispositif selon l'une des revendications 1 à 3,
**caractérisé en ce**
qu'une partie semelle extérieure (114) peut être coupée de manière clabotée et par adhérence à la face inférieure de la partie pied (10).

6. Dispositif selon la revendication 5,
**caractérisé en ce**
que la partie semelle extérieure (114) présente, sur sa face tournée vers la partie pied (10), des éléments d'accrochage rigides (114') qui peuvent être introduits et accrochés dans des ouvertures (110) de la partie pied (10), ainsi qu'un élément d'enclenchement antagoniste (114'') qui coopère avec un lever d'enclenchement (110') positionné en étant élastique dans la partie pied (10), de préférence dans sa partie talon.

7. Dispositif selon la revendication 6,
**caractérisé en ce**
que le levier d'enclenchement (110') est pourvu d'un épaulement d'éjection (110'') pour l'élément semelle (114).

8. Dispositif selon l'une des revendications 1 à 7,
**caractérisé en ce**
que les parties de coque (10, 11, 12, 13) avec leurs différentes parties sont pouvues d'une mutitude de passages (70, 71), répartis sur la surface de la coque, de grandeur et de dimension pouvant être prédéfinie et qu'en particulier de tels passages sont prévus dans la zone des canaux d'aération et de purge de l'air formés par les traverses des coussins.

9. Dispositif selon l'une des revendications 1 à 8,
**caractérisé en ce**
que des canaux de drainage (10') débouchant vers l'extérieur sont prévus au fond de la partie pied (10) dans la zone de la surface de pose du pied pour évacuer le liquide qui s'accumule dans la partie pied.

10. Dispositif selon l'une des revendications 1 à 9,
**caractérisé en ce**
que les coussins (20, 21, 22) sont équipés au moins sur la face tournée vers la partie du corps (6) à envelopper d'une couche sèche qui sert à l'évacuation de liquide et d'une couche de stockage qui recueille l'humidité qui suit cette couche sèche.

11. Dispositif selon l'une des revendications 1 à 10,
**caractérisé en ce**
qu'un insert pour le pied (251) est prévu sur la face intérieure de la partie pied (10), insert qui est configuré comme zone d'un coussin placé sous le pied, où l'on peut faire le vide et qui contient des corps de remplissage.

12. Dispositif selon la revendication 11,
**caractérisé en ce**
que des ouvertures de passage de l'air (251') sont prévues dans le coussin placé sous le pied, ouvertures qui débouchent dans les canaux de drainage (10') de la partie pied (10) et par lesquelles le liquide qui s'est accumulé sous le pied est évacué.

13. Dispositif selon l'une des revendications 1 à 12,
**caractérisé en ce**
que la partie pied (10) et la partie tige (11a) sont reliées l'une à l'autre par des articulations latérales (15) et qu'un dispositif de réglage (3), positionné sur la partie pied (10) et sur la partie tige (11a) est prévu dans la zone du talon, dispositif à l'aide duquel l'angle d'inclinaison de la partie tige (11a) par rapport à la partie pied (10) est réglable.

14. Dispositif selon la revendication 13,
**caractérisé en ce**
que le dispositif de réglage (3) présente un tirant (30), positionné de manière articulée sur la partie pied (10), qui traverse un tuyau fileté (33) vissé dans un écrou de réglage (32) positionné en étant rotatif sur la partie tige (11) et logé dans la partie tige (11a) sans être rotatif mais en étant translatable axialement et qui est pourvu, à son extrémité libre, d'une tête de butée (31) qui coopère avec une butée (33') formée par l'extrémité correspondante du tuyau fileté (33).

15. Dispositif selon la revendication 14,
**caractérisé en ce**
qu'un second écrou de réglage (34) est positionné sur la partie tige (11) en étant rotatif au-dessus du premier écrou de réglage (32) et qu'une tige filetée (35) est vissée dans le second écrou de réglage (34) et est guidée sans être rotative dans la partie tige (11) mais en étant translatable axialement, la face frontale inférieure de la tige filetée (35), tournée vers la tête de butée (31), formant une autre butée (35') pour la tête de butée (31) du tirant (30) si bien qu'une butée réglable individuellement est prévue respectivement au-dessus et au-dessous de la tête de butée (31).

16. Dispositif selon la revendication 14 ou 15,
**caractérisé en ce**
que, pour éviter une manipulation non souhaitée, un blocage de torsion (49, 39), en particulier qui peut être arrêté, est prévu pour l'écrou de réglage (32 et/ou 34).

17. Dispositif selon l'une des revendications 1 à 16,
**caractérisé en ce**
que l'inclinaison latérale de la partie tige (11a) par rapport à la partie pied (10) est réglable transversalement par rapport à l'axe d'articulation (15'), de préférence par des articulations réglables en hauteur (15).

18. Dispositif selon l'une des revendications 1 à 17,
**caractérisé en ce**
que des saillies orientées vers l'intérieur (12''') sont prévues sur la face intérieure de la partie tige (11a) et/ou sur la face intérieure de la coque de recouvrement de la tige (12a) et/ou sur la face intérieure de la coque de recouvrement du pied (13) pour la fixation de la position des coussins.

19. Dispositif selon l'une des revendications 1 à 18,
**caractérisé en ce**
qu'un dispositif de serrage, en particulier une bande de serrage (73 ; 74), est prévu sur la partie pied (10) ou sur la partie tige (11a) dans la zone de l'articulation de l'astragale, dispositif à l'aide duquel le pied et en particulier l'articulation de l'astragale peut être poussé vers l'avant ou vers l'arrière.

20. Dispositif selon l'une des revendications 1 à 19,
**caractérisé en ce**
qu'un élément de serrage, en particulier un étrier de serrage (72) est articulé sur la partie pied (10) ou sur la partie tige (11a) dans la zone de la transition de la partie pied (10) et de la partie tige (11a), en particulier dans la zone adjacente à l'articulation pivotante pour obtenir dans cette zone aussi bien un serrage transversal qu'une pression de la coque de recouvrement de la tige correspondante sur le pied.
